# EUROPÄISCHE PATENTSCHRIFT

(11) **EP 3 813 662 B1**
(45) Veröffentlichungstag und Bekanntmachung des Hinweises auf die Patenterteilung: **18.02.2026**
(21) Anmeldenummer: 19734778.4
(22) Anmeldetag: 28.06.2019
(51) Int. Cl.: A61B 5/11

(54) **VERFAHREN UND SYSTEM ZUR GANGANALYSE**
METHOD AND SYSTEM FOR GAIT ANALYSIS
PROCÉDÉ ET SYSTÈME D'ANALYSE DE LA MARCHE

(30) Priorität: 29.06.2018 EP 18180924
(43) Veröffentlichungstag der Anmeldung: 05.05.2021
(73) Patentinhaber: MCG Motion Capture GmbH, 14471 Potsdam (DE)
(72) Erfinder: ERTELT, Thomas, 12557 Berlin (DE)
(74) Vertreter: Maiwald GmbH
(86) Internationale Anmeldenummer: PCT/EP2019/067401
(87) Internationale Veröffentlichungsnummer: WO 2020/002635

(56) Entgegenhaltungen:
- EP-A2- 0 603 115
- GB-A- 2 549 513
- US-A1- 2009 240 171
- US-A1- 2016 324 445
- THOMAS ERTELT: "Kraftmorphologie der menschlichen Beinbewegung: Dissertation", 2008, XP055533904, Retrieved from the Internet <URL:https://www.researchgate.net/profile/Thomas_Ertelt/publication/259704550_Kraftmorphologie_der_menschlichen_Beinbewegung/links/58e22481aca272059ab13059/Kraftmorphologie-der-menschlichen-Beinbewegung.pdf> [retrieved on 20181213]

## Beschreibung

### Technisches Gebiet

Die Erfindung betrifft ein Verfahren und ein System zur Ganganalyse. Diese kann insbesondere auf einer kinetischen Betrachtung beruhen.

### Technischer Hintergrund

Eine Ganganalyse untersucht die natürliche Fortbewegungsart eines Menschen, nämlich das Gehen und Laufen. Bei einer kinetischen Betrachtung erfolgt dies üblicherweise über eine Messung der Bodenreaktionskraft, die eine Reaktionskraft des Bodens auf die Kraft ist, die der Körper durch die Füße beim Auftreten auf den Boden überträgt.

Die qualitative und/oder quantitative Beschreibung einer Bodenreaktionskraft findet Anwendung in beispielsweise der Medizin, Biomechanik, Robotik usw. Insbesondere in der klinischen Anwendung kann dies herangezogen werden, um Belastungen, Risiken und pathologische Auffälligkeiten bzw. Veränderungen, insbesondere infolge von Krankheiten oder Verletzungen (akut und chronisch), aufzuzeigen. Im sportmedizinischen, wie auch klinischen Bereich dient der Gang zudem als Indikator für mögliche Belastungsrisiken sowie zur Einschätzung und Abklärung vorhandener Beeinträchtigungen von Patienten bzw. als Maß für den Erfolg einer Therapie.

Eine elementare Kenngröße im diagnostischen Bereich stellt dabei der zeitliche Verlauf der Bodenreaktionskraft dar, welcher in der Regel visuell klassifiziert wird und damit hochgradig von der Erfahrung und dem Wissen eines Diagnostikers abhängig ist. Aufgrund der Komplexität des Bodenreaktionskraftverlaufs und zahlreicher Einflussparameter wird neben der Erfahrung des Diagnostikers insbesondere auf einzelne Kennparameter der Bodenreaktionskraft zurückgegriffen. Hierzu zählen u.a. bei der vertikalen Bodenreaktionskraft eine Maximalkraft, ein Zeitpunkt der Maximalkraft, eine Bodenkontaktzeit und bei der horizontalen Bodenreaktionskraft eine maximale Beschleunigung, maximale Verzögerung, eine Netto Beschleunigung usw. Aus diesen Parametern lassen sich weitere Kenngrößen ermitteln wie z.B. Impuls/Kraftstoß, Symmetrien usw. Trotz dieser Kennparameter und Kenngrößen ist es jedoch noch nicht möglich, die Beschreibung des Bodenreaktionskraftverlaufs zu objektivieren, so dass Fehleinschätzungen auftreten können.

Ein Ansatz zur besseren Objektivierung findet sich beispielsweise in Alaqtash, M., Sarkodie-Gyan, T., Yu, H., Fuentes, O., Brower, R. & Abdelgawad, A. (2011). Automatic classification of pathological gait patterns using ground reaction forces and machine learning algorithms. Dabei werden Bodenreaktionskräfte von Schlaganfallpatienten auf Basis der Kennparameter bzw. Kenngrößen durch einen Maschinelles-Lernen-Algorithmus klassifiziert. Nachteilig daran ist, dass nicht der gesamte Bodenreaktionskraftverlauf betrachtet und analysiert wird, so dass das darin für Schlaganfallpatienten vorgeschlagene Verfahren nicht auf andere Felder übertragbar ist.

Thomas Ertelt: "Kraftmorphologie der menschlichen Beinbewegung: Dissertation", 2008, gefunden im Internet: URL: https://www.researchgate.net/profile/Thomas_Ertelt/publication/259704550_Kraftmorphologi e_der_menschlichen_Beinbewegung/links/58e22481aca272059ab13059/Kraftmorphologie-der-menschlichen-Bewegung.pdf, beschreibt ein stationäres System zur Sprunganalyse.

Ganganalyse mittels in Schuhsohlen angeordneter Sensoren sind aus US2009/240171 A1 und US2016/324445 A1 bekannt.

### Zusammenfassung der Erfindung

Die Aufgabe der Erfindung ist es daher, eine verbesserte Möglichkeit zur Ganganalyse mit möglichst hoher Objektivität zur Verfügung zu stellen.

Die Aufgabe wird durch den Gegenstand der unabhängigen Ansprüche gelöst. Vorteilhafte Weiterbildungen der Erfindung ergeben sich aus den abhängigen Ansprüchen, der Beschreibung und den begleitenden Figuren.

Ein erster Aspekt der Erfindung betrifft ein Verfahren zur Ganganalyse, mit den Schritten:
- Erhalten, durch eine in eine Einlegesohle für einen Schuh integrierte elektronische Auswerteeinrichtung, von einen Bodenreaktionskraftverlauf enthaltenden Daten von einer Kraftmesseinrichtung, wobei die Kraftmesseinrichtung durch eine Einlegesohle für einen Schuh mit einer Mehrzahl von Einzelsensoren gebildet ist,
- Bestimmen, durch die Auswerteeinrichtung, einer ersten Näherung des Bodenreaktionskraftverlaufs durch wenigstens eine erste Näherungsfunktion, die durch einen Satz von Koeffizienten definiert ist, und
- Bestimmen, durch die Auswerteeinrichtung, einer zweiten Näherung des Bodenreaktionskraftverlaufs durch eine zweite Näherungsfunktion, die auf Basis des Satzes von Koeffizienten der wenigstens einen ersten Näherungsfunktion gebildet wird; und
- wobei der wenigstens eine Satz von Koeffizienten in einem Koeffizientenpool zusammengefasst wird, aus dem einzelne Koeffizienten zum Bilden der zweiten Näherungsfunktion miteinander kombiniert werden können,
- Bereitstellen eines neuronales Netzes, das dazu eingerichtet ist, die Koeffizienten als Eingangsgröße zu erhalten und eine Klasseninformation als Ausgangsgröße zu erzeugen.

In anderen Worten wird ein gewählter Kraftausschnitt, hier der Bodenreaktionskraftverlauf, angenähert, um diesen gemessenen Verlauf in einer möglichst großen Übereinstimmung möglichst objektiv zu beschreiben.

Mit dieser Konfiguration bietet die Erfindung gleich mehrere Vorteile. So wird eine Möglichkeit geschaffen, die Ganganalyse zu objektivieren und insbesondere eine quantifizierbare Beschreibung unabhängig von einem Diagnostiker zu erreichen. Aus der erfindungsgemäßen Ganganalyse lassen sich einheitliche Interventionen und Strategien ableiten, beispielsweise auch für mögliche chirurgische Eingriffe sowie therapeutische und rehabilitive Maßnahmen.

Der erst Aspekt der Erfindung sieht ja ebenfalls vor, dass der wenigstens eine Satz von Koeffizienten in einem Koeffizientenpool zusammengefasst wird, aus dem einzelne Koeffizienten zum Bilden der zweiten Näherungsfunktion miteinander kombiniert werden.

Je mehr erste Näherungsfunktionen bestimmt werden, desto mehr Sätze von Koeffizienten werden in dem Koeffizientenpool zusammengefasst und stehen für die zweite Näherung zur Verfügung. Durch eine Auswahlmöglichkeit einzelner Koeffizienten aus dem Koeffizientenpool kann der Bodenreaktionskraftverlauf exakt beschrieben werden.

Gemäß einer Weiterbildung kann eine Mehrzahl von zueinander unterschiedlichen ersten Näherungsfunktionen bestimmt werden, die durch den jeweiligen Satz von Koeffizienten definiert sind. Die ersten Näherungsfunktionen können sich z.B. auch nur in einem von mehreren Koeffizienten unterscheiden. Sämtliche Koeffizienten können in dem oben beschriebenen Koeffizientenpool zusammengefasst werden. Durch die Auswahlmöglichkeit aus mehreren Sätzen von Koeffizienten kann die zweite Näherung mit hoher Genauigkeit erfolgen.

In einer anderen Weiterbildung kann vorgesehen sein, dass iterativ wenigstens zwei erste Näherungsfunktionen gewählt werden, aus denen in einem jeweiligen Iterationsschritt eine Summenfunktion und ihre Abweichung vom Bodenreaktionskraftverlauf bestimmt werden, bis die Abweichung ein vorbestimmtes Abbruchkriterium erreicht und daraus die entsprechende erste Näherungsfunktion für die erste Näherung bestimmt wird. Beispielsweise können aus der Summenfunktion der gewählten ersten Näherungsfunktionen Residuen, d.h. die Summe der Abweichungen im Quadrat, bestimmt werden. Durch das Quadrat können kleine Abweichungen in ihrer Wirkung abgeschwächt und große Abweichungen, beispielsweise Abweichungen >1, verstärkt werden. Diese Auswahl bzw. Anpassung kann so oft wiederholt werden, bis ein Minimum der Residuen gegeben ist und die Güte der Funktion ein R² von beispielsweise mindestens 0.8 erreicht. Das Minimum der Residuen, der Werte des R² oder eine Kombination aus beidem können das Abbruchkriterium sein. Die ersten Näherungsfunktionen, die diesem Abbruchkriterium entsprechen, können dann für das Bestimmen der zweiten Näherung durch die zweite Näherungsfunktion herangezogen werden. Dadurch wird mit vergleichsweise geringem Rechenaufwand eine gute erste Näherung als guter Ausgangspunkt für die zweite Näherung erreicht.

Gemäß einer Weiterbildung können die die zweite Näherungsfunktion bildenden Koeffizienten vorab mit wenigstens einem vorbestimmten Datensatz, der eine Mehrzahl von klassifizierten Bodenreaktionskraftverläufen enthalten kann, verglichen werden und daraus validiert werden, ob die Koeffizienten die zweite Näherung ermöglichen. In anderen Worten können die gewählten ersten Näherungsfunktionen bzw. Koeffizienten in einer Art Trainingsset validiert werden. Dazu werden zunächst existierende Bodenreaktionskraftverläufe auf Basis einer z.B. visuellen Analyse voneinander abgegrenzt.

Dabei kann bekannt sein, z.B. durch Beobachtung und einer Zuordnung zu einem Laufstil oder ähnlichem, durch Erkenntnisse aus der Literatur usw., welches Muster eines Bodenreaktionskraftverlaufs einem bestimmten Laufstil entspricht. Es kann dann anhand des Trainingssets geprüft werden, ob es statistisch möglich ist, eine vorbestimmte Klasse, z.B. einen bestimmten Laufstil, tatsächlich zu unterscheiden. So kann z.B. visuell klassifizierten Mustern eine bekannte Kategorie zugeordnet werden, deren Differenzierung in einem Trainingsset geprüft wird.

Eine andere Weiterbildung sieht vor, dass aus den validierten Koeffizienten die zweite Näherungsfunktion iterativ gebildet und die zweite Näherung einer Klasse zugeordnet wird. In anderen Worten, können über den Datensatz Informationen über eine Koeffizientenausprägung in Abhängigkeit zu einer Klassifizierungsgruppe, wie z.B. Laufstil, Sportart, Pathologie ähnliches, erhalten werden. Diese validierten Koeffizienten können nun verwendet werden, um eine möglichst gute Übereinstimmung mit dem zu analysierenden Bodenreaktionskraftverlauf zu erhalten. D.h., dass ein registrierter Bodenreaktionskraftverlauf auf Basis der vorhandenen Koeffizienten approximiert werden kann. Ein Abbruchkriterium kann eine Minimierung des Residuums sein. Es ist nun auch bekannt, welche grundsätzliche Ausprägung die Koeffizienten in einer jeweiligen Klasse aufweisen, so dass für den Bodenreaktionskraftverlauf mit einer entsprechenden Wahrscheinlichkeit die Klasse bestimmt werden kann.

Gemäß einer Weiterbildung kann die erste Näherungsfunktion eine Normalverteilung oder Gauß-Funktion sein. Diese lassen sich einfach bestimmen und weisen eine geringe Komplexität auf. Dennoch kann damit eine gute erste Näherung erreicht werden.

Eine andere Weiterbildung sieht vor, dass die zweite Näherungsfunktion eine Summenfunktion der wenigstens einen ersten Näherungsfunktion sein kann. Diese lässt sich einfach bilden und erlaubt, z.B. über ein Residuum als Abbruchkriterium, eine iterative Näherung mit geringem Rechenaufwand.

In einer Weiterbildung kann der Satz von Koeffizienten einen Maximalwert, einen Mittelwert und/oder eine Breite der ersten Näherungsfunktion umfassen. In anderen Worten kann jede erste Näherungsfunktion drei Koeffizienten umfassen. Dadurch lässt sich beispielsweise eine Normalverteilung oder Gauß-Funktion vollständig definieren.

Gemäß einer anderen Weiterbildung kann eine Anzahl der bestimmten ersten Näherungsfunktion zwischen 1 und 20, bevorzugt zwischen 1 und 15, besonders bevorzugt auf genau 8, festgelegt werden. Die ersten Näherungsfunktionen können beispielsweise durch drei Koeffizienten definiert sein, so dass ich für zwei erste Näherungsfunktionen 2·3=6, für drei erste Näherungsfunktionen 3·3=9 usw. Koeffizienten ergeben. Es hat sich gezeigt, dass für eine klinische Anwendung der Ganganalyse besonders gute Ergebnisse zur Beschreibung des Bodenreaktionskraftverlaufs mit acht ersten Näherungsfunktionen, also 8·3=24 Koeffizienten erreicht werden können. Jedoch können in Abhängigkeit der Komplexität auch 15 oder mehr erste Näherungsfunktionen bestimmt werden. Es hat sich jedoch überraschenderweise gezeigt, dass mit acht ersten Näherungsfunktionen eine gute Qualität bei der Näherung bei vergleichsweise geringem Rechenaufwand erreicht werden kann.

Ein zweiter Aspekt der Erfindung betrifft ein Ganganalysesystem, aufweisend
- eine Kraftmesseinrichtung, die dazu eingerichtet ist, einen Bodenreaktionskraftverlauf zu erfassen, wobei die Kraftmesseinrichtung durch eine Einlegesohle für einen Schuh mit einer Mehrzahl von Einzelsensoren gebildet ist,
- eine elektronische Auswerteeinrichtung, die in eine Einlegesohle für einen Schuh integriert und dazu eingerichtet ist,
- den erfassten Bodenreaktionskraftverlauf durch wenigstens eine erste Näherungsfunktion anzunähern, die durch einen Satz von Koeffizienten definiert ist, und
- den Bodenreaktionskraftverlauf durch eine zweite Näherungsfunktion weiter anzunähern, die auf Basis des Satzes von Koeffizienten gebildet ist; und
- wobei der wenigstens eine Satz von Koeffizienten in einem Koeffizientenpool zusammengefasst wird, aus dem einzelne Koeffizienten zum Bilden der zweiten Näherungsfunktion miteinander kombiniert werden können,
- ferner aufweisend wenigstens ein neuronales Netz, das dazu eingerichtet ist, die Koeffizienten als Eingangsgröße zu erhalten und eine Klasseninformation als Ausgangsgröße zu erzeugen.

In einer Weiterbildung können beispielsweise 8 bis 25, vorzugsweise 12 bis 20, weiter vorzugsweise 14 bis 18, besonders bevorzugt 15 bis 16, Einzelsensoren über eine flächige Erstreckung der Einlegesohle verteilt vorgesehen sein. Die Einzelsensoren können infolge einer darauf wirkenden Kraft Einzelsignale, insbesondere in Form eines Ausschlags und/oder Peaks, bereitstellen, wobei die Summe der Einzelsignale ein Gesamtsignal der Bodenreaktionskraft ergibt. Dieses Gesamtsignal lässt sich beispielsweise durch eine Kombination von Koeffizienten annähern, wie dies im vorstehend beschriebenen Verfahren erläutert ist. Alternativ zu der Kombination von Koeffizienten ist es möglich, lediglich den Ausschlag und/oder Peak, der in etwa vergleichbar mit einer Amplitude einer Gauß-Funktion oder Gauß-Kurve sein kann, und einen Zeitpunkt zu bestimmen, insbesondere einen absoluten oder relativen Zeitpunkt, der in etwa vergleichbar mit einer Lage einer Gauß-Funktion oder Gauß-Kurve sein kann. Eine Breite der Verteilung kann hierbei über eine Bestimmung einer Standardabweichung über die Zeitfunktion bestimmt werden. Hieraus ergeben sich beispielsweise drei Koeffizienten, aus denen eine Näherung bestimmt werden kann. Diese Bestimmung kann aufgrund einer reduzierten Datenmenge beispielsweise direkt durch eine elektronische Auswerteeinrichtung ausgeführt werden, die in die Einlegesohle integriert bzw. eine Baugruppe mit derselben bilden kann.

So wird eine Möglichkeit geschaffen, die Ganganalyse zu objektivieren und insbesondere eine quantifizierbare Beschreibung unabhängig von einem Diagnostiker zu erreichen.

Eine Weiterbildung der Erfindung sieht vor, dass das Ganganalysesystem ferner eine Datenbankeinrichtung aufweisen kann, die dazu eingerichtet sein kann, eine Kombination von die zweite Näherungsfunktion bildenden Koeffizienten einem vorbestimmten Gangverhalten und/oder einer Gangauffälligkeit zuzuordnen. Beispielsweise kann die Datenbankeinrichtung den oben beschriebenen vorbestimmten Datensatz umfassen und dazu eingerichtet sein, dass die Koeffizienten anhand des Datensatzes, insbesondere durch die Auswerteeinrichtung, zu validieren und/oder zu klassifizieren.

Gemäß der Erfindung weist das Ganganalysesystem ferner wenigstens ein neuronales Netz auf, das dazu eingerichtet ist, zumindest die Koeffizienten als Eingangsgröße zu erhalten und eine Klasseninformation als Ausgangsgröße zu erzeugen. In einer Weiterbildung kann zusätzlich das Abbruchkriterium eine weitere Eingangsgröße darstellen. Das neuronale Netz kann mehrschichtig und/oder faltend sein, wobei die Koeffizienten einer Eingangsschicht zugeführt und die Klasseninformation, z.B. Laufstil, Sportart, Pathologie oder ähnliches, von der Ausgangsschicht ausgegeben werden. Das neuronale Netz kann beispielsweise ein Bayesian Regularization Artificial Neural Network (BRANN) umfassen. Dieses bietet eine hohe Robustheit hinsichtlich des Trainings und/oder der Validierung, wobei das neuronale Netz vorzugsweise das Abbruchkriterium für die Näherung berücksichtigen kann. Die Ausgangsgröße kann eine jeweilige Sportart, in einem medizinischen Setting ggf. die jeweilige Pathologie bzw. das Stadium der Beeinträchtigung, sein. Das neuronale Netz kann auch bei sehr komplexen Bodenreaktionskraftverläufen sogar graduelle Unterschiede zwischen den einzelnen Koeffizienten und Mustern erkennen und kann deshalb gegenüber statistischen Verfahren, wie Diskriminanz- oder Clusteranalysen, vorteilhaft sein.

Ein dritter Aspekt der Erfindung betrifft ein Programmelement, das, wenn es mittels eines Prozessors einer elektronischen Auswerteeinrichtung ausgeführt wird, die in eine Einlegesohle für einen Schuh integrierte Auswerteeinrichtung dazu veranlasst, die folgenden Schritte durchzuführen:
- Erhalten von einen Bodenreaktionskraftverlauf enthaltenden Daten von einer Kraftmesseinrichtung, wobei die Kraftmesseinrichtung durch eine Einlegesohle für einen Schuh mit einer Mehrzahl von Einzelsensoren gebildet ist,
- Bestimmen einer ersten Näherung des Bodenreaktionskraftverlaufs durch wenigstens eine erste Näherungsfunktion, die durch einen Satz von Koeffizienten definiert ist, und
- Bestimmen einer zweiten Näherung des Bodenreaktionskraftverlaufs durch eine zweite Näherungsfunktion, die auf Basis des Satzes von Koeffizienten der ersten Näherungsfunktion gebildet wird; und
- wobei der wenigstens eine Satz von Koeffizienten in einem Koeffizientenpool zusammengefasst wird, aus dem einzelne Koeffizienten zum Bilden der zweiten Näherungsfunktion miteinander kombiniert werden können,
- Bereitstellen ein neuronales Netz, das dazu eingerichtet ist, die Koeffizienten als Eingangsgröße zu erhalten und eine Klasseninformation als Ausgangsgröße zu erzeugen.

Die sich daraus ergebenden Vorteile sind vorstehend für das Verfahren sowie das Ganganalysesystem beschrieben.

Ein vierter Aspekt der Erfindung betrifft ein computerlesbares Medium, auf dem das vorstehend beschriebene Programmelement gespeichert ist.

### Kurze Beschreibung der Figuren

Im Folgenden werden bevorzugte Ausführungsformen der Erfindung unter Bezugnahme auf die begleitenden Figuren erläutert. Es zeigen:
- Figur 1: ein erfindungsgemäßes System zur Ganganalyse, das sich zur Durchführung eines erfindungsgemäßen Verfahrens zur Ganganalyse eignet,
- Figur 2: einen beispielhaften Bodenreaktionskraftverlauf mit seiner ersten und zweiten Näherung,
- Figur 3: eine beispielhafte erste Näherungsfunktion, die durch drei Koeffizienten definiert ist, und
- Figur 4: ein Flussdiagramm eines erfindungsgemäßen Verfahrens zur Ganganalyse.

Die Figuren sind lediglich schematische Darstellungen und dienen nur der Erläuterung der Erfindung. Gleiche oder gleichwirkende Elemente sind durchgängig mit den gleichen Bezugszeichen versehen.

### Bevorzugte Ausführungsformen der Erfindung

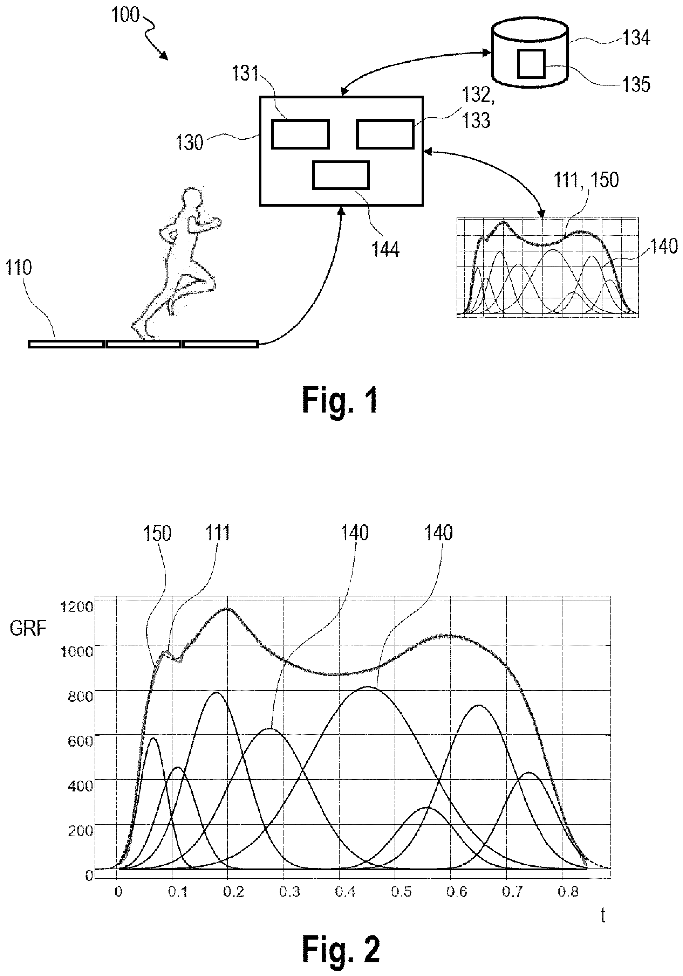

Figur 1 zeigt einen schematischen Aufbau eines Ganganalysesystems 100, das sich insbesondere dazu eignet, Belastungen, Risiken und pathologische Auffälligkeiten bzw. Veränderungen, insbesondere infolge von Krankheiten oder Verletzungen, aufzuzeigen.

Das Ganganalysesystem 100 weist zunächst eine Kraftmesseinrichtung 110 zur messtechnischen Erfassung einer Bodenreaktionskraft über einen Zeitabschnitt, also eines Bodenreaktionskraftverlaufs 111, auf. Die Kraftmesseinrichtung 110 ist hier exemplarisch als Kraftmessplatte ausgeführt und liefert elektronische Daten, die den Bodenreaktionskraftverlauf 111 enthalten. Die Kraftmesseinrichtung 110 kann in einigen Ausführungsformen aber auch durch eine Einlegesohle für einen Schuh gebildet sein, die eine Mehrzahl von Einzelsensoren aufweisen kann. Beispielsweise können 10 bis 25 Einzelsensoren über eine flächige Erstreckung der Einlegesohle verteilt sein, die jeweils ein Einzelsignal, z.B. in Form eines Ausschlags und/oder Peaks, zur Verfügung stellen. Des Weiteren verfügt das Ganganalysesystem 100 über eine elektronische Auswerteeinrichtung 130, die einen Prozessor 131 und einen Speicher 132 aufweist und zum Erhalten der Daten der Kraftmesseinrichtung 110, z.B. über eine Datenschnittstelle, eingerichtet ist. Beispielsweise kann die elektronische Auswerteeinrichtung 130 auch in eine Einlegesohle für einen Schuh integriert sein. In dem Speicher 132 sind Programmanweisungen bzw. ein Programmelement 133 gespeichert, die von dem Prozessor 131 ausgeführt werden können und in denen wenigstens ein Künstliches-Intelligenz-Modul und/oder ein neuronales Netz mit einer Eingangsschicht, einer oder mehreren Zwischenschichten und einer Ausgangsschicht implementiert ist. Vorzugsweise kann das neuronale Netz ein Bayesian Regularization Artificial Neural Network (BRANN) umfassen oder sein. Die Auswerteeinrichtung 130 weist zudem eine Datenbankeinrichtung 134 auf oder ist mit dieser zum Datenaustausch verbunden. In der Datenbankeinrichtung 134 ist wenigstens ein vorbestimmter Datensatz 135 mit Vergleichsdaten zur Auswertung bzw. Klassifizierung des Bodenreaktionskraftverlaufs 111 verfügbar, so dass eine möglichst automatische Auswertung erfolgen kann.

Anhand von Figur 2, die in einem Diagramm einen beispielhaften, von der Kraftmesseinrichtung 110 als Daten gelieferten Bodenreaktionskraftverlauf 111 zeigt, wird im Folgenden ein beispielhafter Betrieb des Ganganalysesystems 100 erläutert. In dem Diagramm stellt die vertikale Achse die Bodenreaktionskraft (engl. ground reaction force) dar, die deshalb mit GRF gekennzeichnet ist. Die horizontale Achse des Diagramms stellt die Zeit dar, die deshalb mit t gekennzeichnet ist.

Zunächst werden die von der Kraftmesseinrichtung 110 erhaltenen Daten auf das Körpergewicht derjenigen Person normiert, deren Gang analysiert werden soll. Dies kann anhand von Gewichtsdaten beispielsweise automatisch oder durch eine Eingabe über eine Benutzerschnittstelle oder ähnliches erfolgen.

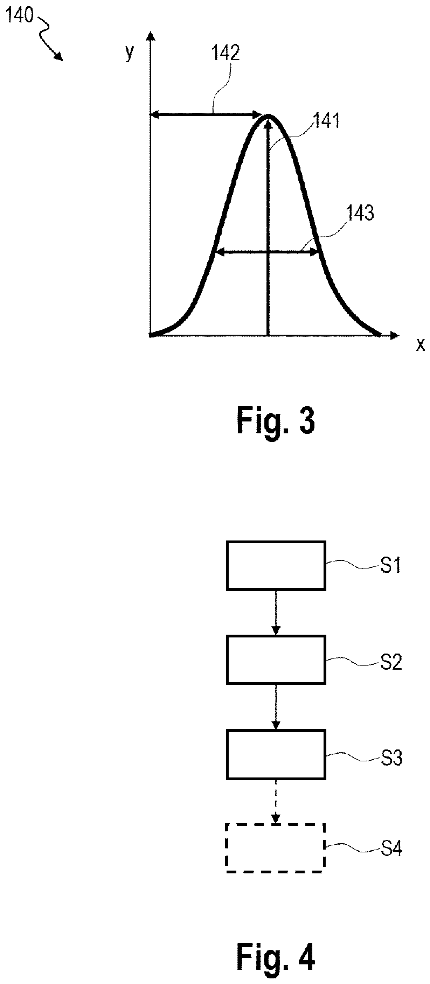

Dann wird mittels der Auswerteeinrichtung 130 eine erste Näherung des Bodenreaktionskraftverlauf 111 bestimmt, wozu mehrere erste Näherungsfunktionen 140 gewählt werden. In dem in Figur 2 gezeigten Ausführungsbeispiel sind für die dortige klinische Ganganalyse genau acht erste Näherungsfunktionen 140 gewählt worden. Die ersten Näherungsfunktionen 140 stellen Normalverteilungen bzw. Gauß-Funktionen dar, unterscheiden sich voneinander und sind durch einen Satz von Koeffizienten 141, 142, 143 definiert, die einen Maximalwert, eine Position, z.B. relativ zu einer X-Achse, insbesondere z.B. die Position eines Maximalwerts und/oder einen Mittelwert, und eine Breite der jeweiligen ersten Näherungsfunktion 140 darstellen (vgl. Figur 3). Die ersten Näherungsfunktionen 140 werden in einem iterativen Verfahren gewählt, indem in jedem Iterationsschritt eine Summenfunktion und ihre Abweichung von dem Bodenreaktionskraftverlauf 111 bestimmt werden, bis die Abweichung ein vorbestimmtes Abbruchkriterium erreicht und daraus die entsprechende erste Näherungsfunktion für die erste Näherung bestimmt wird. In diesem Ausführungsbeispiel werden aus der Summenfunktion der ersten Näherungsfunktionen 140 Residuen, d.h. die Summe der Abweichungen im Quadrat, bestimmt. Sind diese Abweichungen einer ersten Näherungsfunktion 140 zu groß, wird eine andere gewählt. Dies wird vorzugsweise iterativ so oft wiederholt, bis ein Minimum der Residuen gegeben ist und die Güte der Funktion ein R² von beispielsweise mindestens 0.8 erreicht. Vorzugsweise stellt die Kombination aus Minimum der Residuen und die Werte des R² das Abbruchkriterium für das iterative Verfahren dar. Sind die ersten Näherungsfunktionen 140 für die erste Näherung somit gefunden, werden die Koeffizienten 141, 142, 143 aller ersten Näherungsfunktionen 140 in einem Koeffizientenpool 144 (siehe Figur 1) gesammelt und/oder zusammengefasst, wobei in diesem Ausführungsbeispiel dadurch 8 · 3 = 24 Koeffizienten in dem Koeffizientenpool 144 enthalten sind.

Auf Basis der Koeffizienten 141, 142, 143 der ersten Näherungsfunktionen 140, die hier in dem Koeffizientenpool 144 enthalten sind, wird eine zweite Näherung des Bodenreaktionskraftverlaufs 111 durch eine zweite Näherungsfunktion 150 bestimmt. Dies erfolgt durch das Zuführen der Koeffizienten 141, 142, 143 zu der Eingangsschicht des neuronalen Netzes der Auswerteeinrichtung 130. Die zweite Näherungsfunktion 150 ist vorzugsweise eine Summenfunktion der ersten Näherungsfunktionen und ist aus einem Teil oder aus allen Koeffizienten 141, 142, 143 des Koeffizientenpools 144 gebildet. In Figur 2 ist die zweite Näherungsfunktion 150 als gestrichelte Linie eingezeichnet, wobei ein Vergleich mit dem Bodenreaktionskraftverlauf 111 zeigt, dass die zweite Näherung diesen äußerst präzise beschreibt, da diese annähernd deckungsgleich sind. Optional werden die Koeffizienten 141, 142, 143 des Koeffizientenpools 144, die zur Bildung der zweiten Näherungsfunktion 150 herangezogen werden, vorab mit dem Datensatz 135 verglichen, der eine Mehrzahl von bereits klassifizierten Vergleichs-Bodenreaktionskraftverläufen enthält. Anhand dieses Vergleichs wird ermittelt, ob es statistisch möglich ist, eine vorbestimmte Klasse, z.B. einen bestimmten Laufstil, tatsächlich zu unterscheiden. Damit werden die Koeffizienten 141, 142, 143 des Koeffizientenpools 144 vorab validiert. Dann wird aus den anhand des Datensatzes 135 validierten Koeffizienten 141, 142, 143 die zweite Näherungsfunktion 150 in einem iterativen Verfahren gebildet und die zweite Näherung einer Klasse zugeordnet. Dabei werden die Koeffizienten 141, 142, 143 so oft kombiniert, bis ein Abbruchkriterium erreicht wird, das beispielsweise in einer Minimierung eines Residuums bestehen kann. Daraus kann der angenäherte Bodenreaktionskraftverlauf 111 eine Ausprägung der Koeffizienten 141, 142, 143 aufweisen, die einer jeweiligen Klasse zugeordnet werden können. Als Ergebnis wird von der Ausgabeschicht des neuronalen Netzes eine Beschreibung und Klassifikation des Bodenreaktionskraftverlaufs 111 ausgegeben. Dieses Ergebnis lässt eine objektive und valide Ganganalyse zu.

Figur 3 zeigt zur besseren Veranschaulichung eine exemplarische erste Näherungsfunktion, die durch die drei Koeffizienten, nämlich dem Maximalwert 141, der Position, 142 und der Breite 143 definiert ist.

Anhand von Figur 4, die ein Flussdiagramm zeigt, wird nun ein erfindungsgemäßes Verfahren zur Ganganalyse erläutert. Zunächst werden in einem Schritt S1 die Daten, die den Bodenreaktionskraftverlauf 111 enthalten, von der Kraftmesseinrichtung 110 erhalten. Dann wird in einem Schritt S2 eine erste Näherung des Bodenreaktionskraftverlaufs 111 durch eine oder mehrere der ersten Näherungsfunktionen 140 bestimmt, die durch die Koeffizienten 141, 142, 143 definiert sind. In einem Schritt S3 wird eine zweiten Näherung des Bodenreaktionskraftverlaufs 111 durch die zweite Näherungsfunktion 150 bestimmt, die auf Basis der Koeffizienten 141, 142, 143 der einen oder der mehreren ersten Näherungsfunktionen 140 gebildet wird. In einem optionalen Schritt S4 werden die Koeffizienten 141, 142, 143 mit wenigstens einem vorbestimmten Datensatz 135, der eine Mehrzahl von klassifizierten Bodenreaktionskraftverläufen enthält, verglichen. Anhand dieses Vergleichs wird validiert, ob sich die Koeffizienten 141, 142, 143 die zweite Näherung ermöglichen. Weiter optional, werden die Koeffizienten 141, 142, 143 durch den Vergleich mit dem Datensatz 135 einem vorbestimmten Gangverhalten, einer Gangauffälligkeit, und/oder einer anderen Fuß-Boden-Interaktion zugeordnet.

Obwohl die vorliegende Erfindung anhand bevorzugter Ausführungsbeispiele vorstehend beschrieben wurde, ist sie nicht darauf beschränkt, sondern auf vielfältige Art und Weise modifizierbar. Insbesondere lässt sich die vorliegende Erfindung in mannigfaltiger Weise verändern oder modifizieren, ohne vom Kern der Erfindung abzuweichen.

Ergänzend sei darauf hingewiesen, dass "umfassend" und "aufweisend" keine anderen Elemente oder Schritte ausschließt und "eine" oder "ein" keine Vielzahl ausschließt.

Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkungen anzusehen.

### Bezugszeichenliste

- 100: System zur Ganganalyse
- 110: Kraftmesseinrichtung
- 111: Bodenreaktionskraftverlauf
- 130: Auswerteeinrichtung
- 131: Prozessor
- 132: Speicher
- 133: Programmelement
- 134: Datenbankeinrichtung
- 135: vorbestimmter Datensatz
- 140: erste Näherungsfunktion
- 141: erster Koeffizient (z.B. Maximalwert)
- 142: zweiter Koeffizient (z.B. Position)
- 143: dritter Koeffizient (z.B. Breite)
- 144: Koeffizientenpool
- 150: zweite Näherungsfunktion

## Patentansprüche

1. Verfahren zur Ganganalyse, mit den Schritten:
- Erhalten (S1), durch eine in eine Einlegesohle für einen Schuh integrierte elektronische Auswerteeinrichtung (130), von einen Bodenreaktionskraftverlauf (111) enthaltenden Daten von einer Kraftmesseinrichtung (110), wobei die Kraftmesseinrichtung (110) durch eine Einlegesohle für einen Schuh mit einer Mehrzahl von Einzelsensoren gebildet ist,
- Bestimmen (S2), durch die Auswerteeinrichtung (130), einer ersten Näherung des Bodenreaktionskraftverlaufs (111) durch wenigstens eine erste Näherungsfunktion (140), die durch einen Satz von Koeffizienten (141, 142, 143) definiert ist, und
- Bestimmen (S3), durch die Auswerteeinrichtung (130), einer zweiten Näherung des Bodenreaktionskraftverlaufs (111) durch eine zweite Näherungsfunktion (150), die auf Basis des Satzes von Koeffizienten (141, 142, 143) der wenigstens einen ersten Näherungsfunktion (140) gebildet wird; und
- wobei der wenigstens eine Satz von Koeffizienten (141, 142, 143) in einem Koeffizientenpool (144) zusammengefasst wird, aus dem einzelne Koeffizienten (141, 142, 143) zum Bilden der zweiten Näherungsfunktion (150) miteinander kombiniert werden können,
- Bereitstellen eines neuronales Netzes, das dazu eingerichtet ist, die Koeffizienten (141, 142, 143) als Eingangsgröße zu erhalten und eine Klasseninformation als Ausgangsgröße zu erzeugen.

2. Verfahren nach Anspruch 1, wobei eine Mehrzahl von zueinander unterschiedlichen ersten Näherungsfunktionen (140) bestimmt wird, die durch den jeweiligen Satz von Koeffizienten (141, 142, 143) definiert sind.

3. Verfahren nach einem der vorhergehenden Ansprüche, wobei iterativ wenigstens zwei erste Näherungsfunktionen (140) gewählt werden, aus denen in einem jeweiligen Iterationsschritt eine Summenfunktion und ihre Abweichung vom Bodenreaktionskraftverlauf (111) bestimmt werden, bis die Abweichung ein vorbestimmtes Abbruchkriterium erreicht und daraus die entsprechende erste Näherungsfunktion (140) für die erste Näherung bestimmt wird.

4. Verfahren nach einem der vorhergehenden Ansprüche, wobei die die zweite Näherungsfunktion (150) bildenden Koeffizienten (141, 142, 143) vorab mit wenigstens einem vorbestimmten Datensatz (135), der eine Mehrzahl von klassifizierten Bodenreaktionskraftverläufen enthält, verglichen (S4) werden und daraus validiert wird, ob die Koeffizienten (141, 142, 143) die zweite Näherung ermöglichen.

5. Verfahren nach Anspruch 4, wobei aus den validierten Koeffizienten (141, 142, 143) die zweite Näherungsfunktion (150) iterativ gebildet und die zweite Näherung einer Klasse zugeordnet wird.

6. Verfahren nach einem der vorhergehenden Ansprüche, wobei die erste Näherungsfunktion (140) eine Normalverteilung oder Gauß-Funktion ist.

7. Verfahren nach einem der vorhergehenden Ansprüche, wobei die zweite Näherungsfunktion (150) eine Summenfunktion der wenigstens einen ersten Näherungsfunktion (140) ist.

8. Verfahren nach einem der vorhergehenden Ansprüche, wobei eine Anzahl der bestimmten ersten Näherungsfunktion (140) zwischen 1 und 20, bevorzugt zwischen 1 und 15, besonders bevorzugt auf genau 8, festgelegt wird.

9. Ganganalysesystem (100), aufweisend
- eine Kraftmesseinrichtung (110), die dazu eingerichtet ist, einen Bodenreaktionskraftverlauf (111) zu erfassen, wobei die Kraftmesseinrichtung (110) durch eine Einlegesohle für einen Schuh mit einer Mehrzahl von Einzelsensoren gebildet ist,
- eine elektronische Auswerteeinrichtung (130), die in eine Einlegesohle für einen Schuh integriert und dazu eingerichtet ist,
- den erfassten Bodenreaktionskraftverlauf (111) durch wenigstens eine erste Näherungsfunktion (140) anzunähern, die durch einen Satz von Koeffizienten (141, 142, 143) definiert ist, und
- den Bodenreaktionskraftverlauf (111) durch eine zweite Näherungsfunktion (150) weiter anzunähern, die auf Basis des Satzes von Koeffizienten (141, 142, 143) gebildet ist; und
- wobei der wenigstens eine Satz von Koeffizienten (141, 142, 143) in einem Koeffizientenpool (144) zusammengefasst wird, aus dem einzelne Koeffizienten (141, 142, 143) zum Bilden der zweiten Näherungsfunktion (150) miteinander kombiniert werden können,
- ferner aufweisend wenigstens ein neuronales Netz, das dazu eingerichtet ist, die Koeffizienten (141, 142, 143) als Eingangsgröße zu erhalten und eine Klasseninformation als Ausgangsgröße zu erzeugen.

10. Ganganalysesystem (100) nach Anspruch 9, ferner aufweisend eine Datenbankeinrichtung (134), die dazu eingerichtet ist, eine Kombination von die zweite Näherungsfunktion (150) bildenden Koeffizienten (141, 142, 143) einem vorbestimmten Gangverhalten, einer Gangauffälligkeit, und/oder einer anderen Fuß-Boden-Interaktion zuzuordnen.

11. Ganganalysesystem (100) nach Anspruch 10, wobei die Datenbankeinrichtung (134) in eine Einlegesohle für einen Schuh integriert ist.

12. Programmelement (132), das, wenn es mittels eines Prozessors (131) einer elektronischen Auswerteeinrichtung (130) ausgeführt wird, die in eine Einlegesohle für einen Schuh integrierte Auswerteeinrichtung (130) dazu veranlasst, die folgenden Schritte durchzuführen:
- Erhalten von einen Bodenreaktionskraftverlauf (111) enthaltenden Daten von einer Kraftmesseinrichtung (110), wobei die Kraftmesseinrichtung (110) durch eine Einlegesohle für einen Schuh mit einer Mehrzahl von Einzelsensoren gebildet ist,
- Bestimmen einer ersten Näherung des Bodenreaktionskraftverlaufs (111) durch wenigstens eine erste Näherungsfunktion (140), die durch einen Satz von Koeffizienten (141, 142, 143) definiert ist, und
- Bestimmen einer zweiten Näherung des Bodenreaktionskraftverlaufs (111) durch eine zweite Näherungsfunktion (150), die auf Basis des Satzes von Koeffizienten (141, 142, 143) der ersten Näherungsfunktion (140) gebildet wird; und
- wobei der wenigstens eine Satz von Koeffizienten (141, 142, 143) in einem Koeffizientenpool (144) zusammengefasst wird, aus dem einzelne Koeffizienten (141, 142, 143) zum Bilden der zweiten Näherungsfunktion (150) miteinander kombiniert werden können,
- Bereitstellen ein neuronales Netz, das dazu eingerichtet ist, die Koeffizienten (141, 142, 143) als Eingangsgröße zu erhalten und eine Klasseninformation als Ausgangsgröße zu erzeugen.

13. Computerlesbares Medium, auf dem ein Programmelement (132) nach Anspruch 12 gespeichert ist.

## Claims

1. A method for gait analysis, comprising:
- obtaining (S1), by an electronic evaluation device (130) integrated in an insole for a shoe, data including a ground reaction force curve (111) from a force measuring device (110), wherein the force measuring device (110) is formed by an insole for a shoe having a plurality of single sensors;
- determining (S2), by the evaluation device (130), a first approximation of the ground reaction force curve (111) by at least a first approximation function (140) defined by a set of coefficients (141, 142, 143), and
- determining (S3), by the evaluation device (130), a second approximation of the ground reaction force curve (111) by a second approximation function (150) formed on the basis of the set of coefficients (141, 142, 143) of the at least one first approximation function (140), and
- wherein the at least one set of coefficients (141, 142, 143) is combined into a coefficient pool (144) from which individual coefficients (141, 142, 143) can be combined to form the second approximation function (150),
- providing a neural network which is configured to receive the coefficients (141, 142, 143) as an input variable and to generate a class information as an output variable.

2. Method of claim 1, wherein a plurality of mutually different first approximation functions (140) defined by the respective set of coefficients (141, 142, 143) are determined.

3. Method according to any one of the preceding claims, wherein at least two first approximate functions (140) are iteratively selected, from which a sum function and its deviation from the ground reaction force curve (111) are determined in a respective iteration step until the deviation reaches a predetermined termination criterion and the corresponding first approximate function (140) for the first approximation is determined therefrom.

4. Method according to any one of the preceding claims, wherein the coefficients (141, 142, 143) forming the second approximation function (150) are compared (S4) in advance with at least one predetermined data set (135) comprising a plurality of classified ground reaction force trajectories and validated therefrom whether the coefficients (141, 142, 143) enable the second approximation.

5. Method according to claim 4, wherein the second approximation function (150) is iteratively formed from the validated coefficients (141, 142, 143) and the second approximation is assigned to a class.

6. Method according to any one of the preceding claims, wherein the first approximation function (140) is a normal distribution or Gaussian function.

7. Method according to any one of the preceding claims, wherein the second approximation function (150) is a sum function of the at least one first approximation function (140).

8. Method according to any one of the preceding claims, wherein a number of the determined first approximation function (140) is set between 1 and 20, preferably between 1 and 15, more preferably to exactly 8.

9. A Gait analysis system (100), comprising
- a force measuring device (110) configured to detect a ground reaction force (111), wherein the force measuring device (110) is formed by an insole for a shoe having a plurality of single sensors,
- an electronic evaluation device (130), integrated in an insole for a shoe and configured to:
- approximate the detected ground reaction force curve (111) by at least a first approximation function (140) defined by a set of coefficients (141, 142, 143), and
- further approximate the ground reaction force curve (111) by a second approximation function (150) formed on the basis of the set of coefficients (141, 142, 143)
- wherein the at least one set of coefficients (141, 142, 143) is combined into a coefficient pool (144) from which individual coefficients (141, 142, 143) can be combined to form the second approximation function (150),
- further comprising a neural network which is configured to receive the coefficients (141, 142, 143) as an input variable and to generate a class information as an output variable.

10. Gait analysis system (100) according to claim 9, further comprising a database (134) adapted to associate a combination of coefficients (141, 142, 143) forming the second approximation function (150) with a predetermined gait, a gait abnormality, and/or another foot-ground interaction.

11. Gait analysis system (100) according to claim 10, wherein the database (134) is integrated into an insole for a shoe.

12. A program element (132) which, when executed by means of a processor (131) of an electronic evaluation device (130), causes the evaluation device (130) integrated into an insole for a shoe to perform the following steps:
- obtaining data including a ground reaction force curve (111) from a force measuring device (110), wherein the force measuring device (110) is formed by an insole for a shoe having a plurality of single sensors,
- determining a first approximation of the ground reaction force curve (111) by at least a first approximation function (140) defined by a set of coefficients (141, 142, 143), and
- determining a second approximation of the ground reaction force curve (111) by a second approximation function (150) formed on the basis of the set of coefficients (141, 142, 143) of the first approximation function (140), and
- wherein the at least one set of coefficients (141, 142, 143) is combined into a coefficient pool (144) from which individual coefficients (141, 142, 143) can be combined with each other to form the second approximation function (150),
- providing a neural network which is configured to receive the coefficients (141, 142, 143) as an input variable and to generate a class information as an output variable.

13. A computer readable medium on which is stored a program element (132) according to claim 12.

## Revendications

1. Procédé d'analyse de la marche, comportant les étapes consistant à :
- obtenir (S1), par un dispositif d'évaluation électronique (130) intégré dans une semelle intérieure d'une chaussure, des données comprenant une courbe de force de réaction du sol (111) provenant d'un dispositif de mesure de force (110), dans lequel le dispositif de mesure de force (110) est formé par une semelle intérieure d'une chaussure comportant une pluralité de capteurs individuels,
- déterminer (S2), par le dispositif d'évaluation (130), une première approximation de la courbe de force de réaction du sol (111) par au moins une première fonction d'approximation (140) qui est définie par un ensemble de coefficients (141, 142, 143), et
- déterminer (S3), par le dispositif d'évaluation (130), une seconde approximation de la courbe de force de réaction du sol (111) par une seconde fonction d'approximation (150) qui est formée sur la base de l'ensemble de coefficients (141, 142, 143) de ladite au moins une première fonction d'approximation (140) ; et
- dans lequel ledit au moins un ensemble de coefficients (141, 142, 143) est réuni dans un groupe de coefficients (144) à partir duquel des coefficients individuels (141, 142, 143) peuvent être combinés les uns avec les autres pour former la seconde fonction d'approximation (150),
- fournir un réseau neuronal qui est configuré pour obtenir les coefficients (141, 142, 143) en tant que grandeur d'entrée et pour générer une information concernant une classe en tant que grandeur de sortie.

2. Procédé selon la revendication 1, dans lequel une pluralité de premières fonctions d'approximation (140) différentes les unes des autres est déterminée, lesquelles étant définies par l'ensemble de coefficients (141, 142, 143) respectif.

3. Procédé selon l'une des revendications précédentes, dans lequel au moins deux premières fonctions d'approximation (140) sont choisies de manière itérative, à partir desquelles une fonction de sommation et son écart par rapport à la courbe de force de réaction du sol (111) sont déterminés dans une étape d'itération respective, jusqu'à ce que l'écart atteigne un critère d'arrêt prédéterminé et que la première fonction d'approximation (140) correspondante pour la première approximation soit déterminée à partir de celle-ci.

4. Procédé selon l'une des revendications précédentes, dans lequel les coefficients (141, 142, 143) formant la seconde fonction d'approximation (150) sont préalablement comparés (S4) à au moins un ensemble de données (135) prédéterminé qui comprend une pluralité de courbes de force de réaction du sol classées et, à partir de cette comparaison, il est validé si les coefficients (141, 142, 143) permettent la seconde approximation.

5. Procédé selon la revendication 4, dans lequel la seconde fonction d'approximation (150) est formée de manière itérative à partir des coefficients (141, 142, 143) validés et une classe est associée à la seconde approximation.

6. Procédé selon l'une des revendications précédentes, dans lequel la première fonction d'approximation (140) est une distribution normale ou une fonction gaussienne.

7. Procédé selon l'une des revendications précédentes, dans lequel la seconde fonction d'approximation (150) est une fonction de sommation de ladite au moins une première fonction d'approximation (140).

8. Procédé selon l'une des revendications précédentes, dans lequel un nombre de la première fonction d'approximation (140) déterminée est fixé entre 1 et 20, de manière préférée entre 1 et 15, de manière particulièrement préférée exactement à 8.

9. Système d'analyse de la marche (100), comportant :
- un dispositif de mesure de force (110) qui est configuré pour détecter une courbe de force de réaction du sol (111), dans lequel le dispositif de mesure de force (110) est formé par une semelle intérieure d'une chaussure comportant une pluralité de capteurs individuels,
- un dispositif d'évaluation électronique (130) qui est intégré dans une semelle intérieure d'une chaussure et est configuré pour :
- approximer la courbe de force de réaction du sol (111) détectée, par au moins une première fonction d'approximation (140) qui est définie par un ensemble de coefficients (141, 142, 143), et
- approximer en outre la courbe de force de réaction du sol (111) par une seconde fonction d'approximation (150) qui est formée sur la base de l'ensemble de coefficients (141, 142, 143) ; et
- dans lequel ledit au moins un ensemble de coefficients (141, 142, 143) est réuni dans un groupe de coefficients (144) à partir duquel des coefficients individuels (141, 142, 143) peuvent être combinés les uns avec les autres pour former la seconde fonction d'approximation (150),
- comportant en outre au moins un réseau neuronal qui est configuré pour obtenir les coefficients (141, 142, 143) en tant que grandeur d'entrée et pour générer une information concernant une classe en tant que grandeur de sortie.

10. Système d'analyse de la marche (100) selon la revendication 9, comportant en outre un dispositif de base de données (134) qui est conçu pour associer une combinaison de coefficients (141, 142, 143) formant la seconde fonction d'approximation (150) à un comportement de marche prédéterminé, à une anomalie de marche et/ou à une autre interaction pied-sol.

11. Système d'analyse de la marche (100) selon la revendication 10, dans lequel le dispositif de base de données (134) est intégré dans une semelle intérieure d'une chaussure.

12. Élément de programme (132) qui, lorsqu'il est exécuté au moyen d'un processeur (131) d'un dispositif d'évaluation électronique (130), amène le dispositif d'évaluation (130) intégré dans une semelle intérieure d'une chaussure à réaliser les étapes suivantes :
- obtenir des données comprenant une courbe de force de réaction du sol (111) provenant d'un dispositif de mesure de force (110), dans lequel le dispositif de mesure de force (110) est formé par une semelle intérieure d'une chaussure comportant une pluralité de capteurs individuels,
- déterminer une première approximation de la courbe de force de réaction du sol (111) par au moins une première fonction d'approximation (140) qui est définie par un ensemble de coefficients (141, 142, 143), et
- déterminer une seconde approximation de la courbe de force de réaction du sol (111) par une seconde fonction d'approximation (150) qui est formée sur la base de l'ensemble de coefficients (141, 142, 143) de la première fonction d'approximation (140) ; et
- dans lequel ledit au moins un ensemble de coefficients (141, 142, 143) est réuni dans un groupe de coefficients (144) à partir duquel des coefficients individuels (141, 142, 143) peuvent être combinés les uns avec les autres pour former la seconde fonction d'approximation (150),
- fournir un réseau neuronal qui est configuré pour obtenir les coefficients (141, 142, 143) en tant que grandeur d'entrée et pour générer une information concernant une classe en tant que grandeur de sortie.

13. Support lisible par ordinateur sur lequel est stocké un élément de programme (132) selon la revendication 12.
